# FASCICULE DE BREVET EUROPEEN

(11) **EP 0 633 317 B1**
(45) Date de publication et mention de la délivrance du brevet: **23.03.2005**
(21) Numéro de dépôt: 94420177.1
(22) Date de dépôt: 23.06.1994
(51) Int. Cl.: C12N 15/82, C12N 15/29, C12N 5/00, C12N 1/21, A01H 5/00

(54) **Séquence d'ADN isolée pouvant servir de zone terminatrice dans un gène chimère utilisable pour la transformation des plantes**
Isolierte DNA-Sequenz, die als Terminatorregion in chimären Genen zur Transformation von Pflanzen eingesetzt werden kann
Isolated DNA sequence able to function as a termination region in chimeric genes for plant transformation

(30) Priorité: 25.06.1993 FR 9308029
(43) Date de publication de la demande: 11.01.1995
(73) Titulaire: Bayer CropScience S.A., 69009 Lyon (FR)
(72) Inventeur: Atanassova, Rossitza, F-67000 Strasbourg (FR); De Rose, Richard, F-69009 Lyon (FR); Freyssinet, Georges, F-69450 St. Cyr au Mont d'Or (FR); Gigot, Claude, F-67000 Strasbourg (FR); Lebrun, Michel, F-69009 Lyon (FR)
(74) Mandataire: Tetaz, Franck

(56) Documents cités:
- EP-A- 0 507 698
- GENE, vol.71, 1988, AMSTERDAM NL pages 217 - 223 CHABOUTE, M.-E., ET AL. 'Polyadenylation of histone H3 and H4 mRNAs in dicotyledonous plants'
- PLANT MOLECULAR BIOLOGY., vol.8, 1987, DORDRECHT, THE NETHERLANDS. pages 179 - 191 CHABOUTE, M.E., ET AL. 'Genomic organization and nucleotide sequences of two histone H3 and two histone H4 genes of Arabidopsis thaliana'
- BIOLOGICAL ABSTRACTS, vol. 88 1989, Philadelphia, PA, US; abstract no. 119487, NAKAYAMA, T., ET AL. 'Cis-acting sequences that modulate transcription of wheat histone H3 gene and 3' processing of H3 premature messenger RNA' & PLANT CELL PHYSIOL., vol.30, no.6, 1989 pages 825 - 832
- THE PLANT JOURNAL, vol.2, no.3, 1992 pages 291 - 300 ATANASSOVA, R., ET AL. 'A 126 bp fragment of a plant histone gene promoter confers preferential expression in meristems of transgenic Arabidopsis'
- MOL. GEN. GENET., vol.231, 1992 pages 276 - 285 LEPETIT, M., ET AL. 'A plant histone gene promoter can direct both replication-dependent and -independent gene expression in transgenic plants'
- NUCLEIC ACIDS RESEARCH, vol.16, no.4, 1988 pages 1295 - 1304 CHAUBET, N., ET AL. 'The histone H3 and H4 mRNAs are polyadenylated in maize'

## Description

La présente invention concerne l'utilisation de zone terminatrices isolées de gènes transcrits de plantes, de nouveaux gènes chimères les contenant et leur utilisation pour la transformation des plantes.

De nombreux caractères phénotypiques associés à l'expression d'un ou quelques éléments géniques peuvent être intégrés dans le génome de plantes et conférer ainsi à ces plantes transgéniques des propriétés agronomiques avantageuses. De façon non extensive on peut citer: les résistances à des agents pathogènes des cultures, la résistance à des produits phytosanitaires phytotoxiques, la production de substances à intérêt alimentaire ou pharmacologique. En plus de l'isolement et la caractérisation des éléments géniques codant pour ces différents caractères, une expression appropriée doit être assurée. Cette expression appropriée peut se situer aussi bien au niveau qualitatif que quantitatif. Au niveau qualitatif, par exemple spatial: expression préférentielle dans un tissu particulier, ou temporel: expression inductible. Au niveau quantitatif par la quantité accumulée du produit d'expression du gène introduit. Cette expression appropriée dépend pour une large part de la présence d'éléments géniques de régulation associés aux transgènes, en particulier pour ce qui concerne les éléments quantitatifs et qualitatifs. Parmi les éléments primordiaux assurant cette régulation appropriée, l'utilisation de zones promotrices homologues ou hétérologues, simples ou combinées a été largement décrite dans la littérature scientifique. L'utilisation de zone terminatrice en aval du transgène ont été utilisées à seule fin de mettre une borne permettant d'arrêter le processus de transcription du transgène, sans présupposé quant à leur rôle sur la qualité ou la quantité de l'expression du transgène.

La présente invention concerne des gènes chimères contenant des zones terminatrices de gènes transcrits de plantes, en particulier des zones terminatrices de gènes d'histone végétal, et leur utilisation pour la transformation des plantes. Elle concerne plus particulièrement l'utilisation conjointe de zones terminatrices et de promoteurs isolés d'un même gène transcrit de plantes. Elle permet l'expression appropriée à la fois quantitative et qualitative des transgènes sous le contrôle de ces éléments de régulation génique. Cette expression appropriée obtenue par l'utilisation de la présente invention peut concerner des caractères tels que: la résistance à des agents pathogènes des cultures, la résistance à des produits phytosanitaires phytotoxiques, la production de substances à intérêt alimentaire ou pharmacologique. En particulier, elle permet de conférer aux plantes transgéniques une tolérance accrue à des herbicides par une expression préférentielle, qualitative et quantitative, du produit d'expression des gènes chimères dans les régions de la plante en croissance rapide. Cette expression appropriée particulière du gène de résistance herbicide est obtenue par utilisation conjointe des éléments de régulation promoteur et zone terminatrice du gène d'histone H4A748 d'*Arabidopsis thaliana*. Un tel profil d'expression peut être obtenu pour tous les caractères présentant un intérêt, tels que décrits ci-dessus, avec les éléments de régulation utilisés pour conférer une tolérance herbicide accrue. La présente invention concerne également les cellules végétales transformées à l'aide de ces gènes et les plantes transformées régénérées à partir de ces cellules ainsi que les plantes issues de croisements utilisant ces plantes transformées.

Parmi les produits phytosanitaires utilisés pour la protection des cultures, les produits systémiques sont caractérisés en ce qu'ils sont véhiculés dans la plante après application et, pour certain d'entre eux, s'accumulent dans les parties en croissance rapide, notamment les apex caulinaires et racinaires, provoquant, dans le cas des herbicides, l'altération, jusqu'à la destruction, des plantes sensibles. Pour certain des herbicides présentant ce type de comportement, le mode d'action primaire est connu et résulte d'une inactivation d'enzymes caractérisées impliquées dans des voies de biosynthèse de composés nécessaires au bon développement des plantes cibles. Les enzymes cibles de ces produits peuvent être localisées dans différents compartiments subcellulaire et l'observation du mode d'action de produits connus montre le plus souvent une localisation dans le compartiment plastidial.

La tolérance des plantes sensibles à un produit appartenant à ce groupe d'herbicides, et dont la cible primaire est connue, peut-être obtenue par introduction stable dans leur génome d'un gène codant pour l'enzyme cible, d'origine phylogénétique quelconque, mutée ou non quant aux caractéristiques d'inhibition par l'herbicide du produit de l'expression de ce gène. Une autre approche consiste à introduire de façon stable dans le génome des plantes sensibles un gène d'origine phylogénétique quelconque codant pour une enzyme capable de réaliser une métabolisation de l'herbicide en un composé inactif et non toxique pour le développement de la plante. Dans ce dernier cas, il n'est pas nécessaire d'avoir caractérisé la cible de l'herbicide.

Etant donné le mode de distribution et d'accumulation des produits de ce type dans les plantes traitées, il est intéressant de pouvoir exprimer le produit de la traduction de ces gènes de façon à permettre leur expression préférentielle et leur accumulation dans les régions de la plante en croissance rapide où ces produits s'accumulent. De plus, et dans le cas où la cible de ces produits est localisée dans un compartiment cellulaire autre que le cytoplasme, il est intéressant de pouvoir exprimer le produit de la traduction de ces gènes sous forme d'un précurseur contenant une séquence polypeptidique permettant l'adressage de la protéine conférant la tolérance dans le compartiment adéquat, et en particulier dans le compartiment plastidial.

A titre d'exemple illustrant cette approche on peut citer le glyphosate, le sulfosate ou la fosamétine qui sont des herbicides systémiques à large spectre de la famille des phosphonométhylglycines. Ils agissent essentiellement comme inhibiteurs compétitifs vis à vis du PEP (phosphoénolpyruvate) de la 5-énolpyruvylshikimate-3-phosphate synthase (EPSPS, EC 2.5.1.19). Après leur application sur la plante, ils sont véhiculés dans la plante où ils s'accumulent dans les parties en croissance rapide, notamment les apex caulinaires et racinaires, provoquant l'altération, jusqu'à la destruction, des plantes sensibles.

L'EPSPS, cible principale de ces produits est une enzyme de la voie de biosynthèse des acides aminés aromatiques localisée dans le compartiment plastidial. Cette enzyme est codée par un ou des gènes nucléaires et synthétisée sous forme d'un précurseur cytoplasmique puis importée dans les plastes où elle s'accumule sous sa forme mature.

La tolérance des plantes au glyphosate et aux produits de la famille est obtenue par l'introduction stable dans leur génome d'un gène d'EPSPS d'origine végétale ou bactérienne, mutée ou non quant aux caractéristiques d'inhibition par le glyphosate du produit de ce gène. Etant donné le mode d'action du glyphosate, il est intéressant de pouvoir exprimer le produit de la traduction de ce gène de façon à permettre son accumulation importante dans les plastes et de plus, dans les régions de la plante en croissance rapide où les produits s'accumulent.

Il est connu, par exemple d'après le brevet américain 4 535 060, de conférer à une plante une tolérance à un herbicide du type ci-dessus, en particulier la N-phosphonomethylglycine ou glyphosate, par introduction dans le génome des plantes d'un gène codant pour une EPSPS portant au moins une mutation rendant cette enzyme plus résistante à son inhibiteur compétitif (le glyphosate), après localisation de l'enzyme dans le compartiment plastidial. Ces techniques demandent cependant à être améliorées pour une plus grande fiabilité dans l'emploi de ces plantes lors d'un traitement par ces produits dans des conditions agronomiques.

Dans la présente description, on entend par "plante" tout organisme multicellulaire différentié capable de photosynthèse et par "cellule végétale" toute cellule issue d'une plante et pouvant constituer des tissus indifférenciés tels que des cals, ou des tissus différentiés tels que des embryons ou des parties de plantes ou des plantes ou des semences. On entend par "zone terminatrice" une séquence d'ADN isolée de longueur variable, située en aval de la partie codante, et qui signale la fin de la transcription. On entend par gène de tolérance à un herbicide tout gène, d'origine phylogénétique quelconque, codant soit pour l'enzyme cible de l'herbicide, présentant ou non une ou des mutations quant aux caractéristiques d'inhibition par l'herbicide, soit pour une enzyme capable de métaboliser l'herbicide en un composé inactif et non toxique pour la plante. On entend par zones de la plante en croissance rapide, les régions qui sont le siège de multiplications cellulaires importantes, en particulier les régions apicales.

La présente invention concerne la production de plantes transformées ayant notamment une tolérance accrue à des herbicides s'accumulant dans les zones en croissance rapides des plantes traitées, par régénération de cellules transformées à l'aide de nouveaux gènes chimères comportant un gène de tolérance à ces produits. L'invention a également pour objet la production de plantes transformées ayant une tolérance accrue aux herbicides de la famille des phosphonométhylglycines par régénération de cellules transformées à l'aide de nouveaux gènes chimères comportant un gène de tolérance à ces herbicides. L'invention concerne également ces nouveaux gènes chimères, ainsi que des plantes transformées plus tolérantes en raison d'une meilleure tolérance dans les parties en croissance rapide de ces plantes, ainsi que les plantes issues de croisements utilisant ces plantes transformées. Plus particulièrement, l'invention a pour objet un gène chimère comprenant dans le sens de la transcription une zone promotrice, une séquence codant pour une protéine d'intérêt agronomique et une zone terminatrice, caractérisé en ce que la zone terminatrice est une zone terminatrice d'un gène d'histone végétal.

Selon un mode particulier de réalisation, l'invention a pour objet un gène chimère pour conférer aux plantes notamment une tolérance accrue vis à vis d'un herbicide ayant pour cible l'EPSPS, comprenant, dans te sens de la transcription, une zone promotrice, une zone peptide de transit, une séquence codant pour une enzyme de tolérance aux produits de la famille des phosphonométhylglycines et une zone terminatrice, caractérisé en ce que la zone terminatrice est constituée d'un fragment d'une zone terminatrice d'un gène d'histone végétale dans une orientation quelconque relativement à son orientation initiale dans le gène duquel il dérive, permettant l'expression préférentielle et l'accumulation de la protéine de tolérance à l'herbicide dans les zones d'accumulation du dit herbicide.

Le gène d'histone, dont est issu le zone terminatrice selon l'invention, provient d'une plante monocotylédone telle que par exemple le blé, le maïs ou le riz ou de préférence d'une plante dicotylédone telle que par exemple la luzerne, le tournesol, le soja, le colza ou de préférence *Arabidopsis thaliana*. On utilise de préférence un gène d'histone du type H3 ou de préférence du type H4.

La zone peptide de transit comprend, dans le sens de la transcription, au moins un peptide de transit d'un gène végétal codant pour une enzyme à localisation plastidiale, une partie de la séquence de la partie mature N-terminale d'un gène végétal codant pour une enzyme à localisation plastidiale, puis un second peptide de transit d'un gène végétal codant pour une enzyme à localisation plastidiale, de préférence le gène de la petite sous-unité de la ribulose-1,5-bisphosphate carboxylase/oxygénase (RuBisCO) selon la demande de brevet européen 508 909. Cette zone caractéristique a comme rôle de permettre le relargage dans le compartiment plastidial d'un polypeptide mature avec une efficacité maximale, de préférence sous forme native.

La séquence codante utilisable dans le gène chimère selon l'invention provient d'un gène de tolérance herbicide d'origine phylogénétique quelconque. Cette séquence peut être notamment celle de l'EPSPS mutée ayant un degré de tolérance au glyphosate.

La zone promotrice selon la demande de brevet européen 507 698 peut être d'origine quelconque, sous forme simple ou dupliquée ou combinée d'un gène s'exprimant naturellement dans les plantes, c'est à dire, par exemple bactérienne telle que celle du gène de la nopaline synthase, ou virale telle que celle du transcrit 35S du virus de la mosaïque du chou-fleur, ou de préférence végétale telle que celle de la petite sous-unité de la ribulose-1,5-bisphosphate carboxylase/oxygénase ou de préférence telle que celle d'un gène d'histone végétale et de préférence d'*Arabidopsis thaliana*. On utilise de préférence un gène d'histone du type H3 ou de préférence H4.

Le gène chimère selon l'invention peut comprendre, en plus des parties essentielles ci-dessus, une zone intermédiaire non traduite (linker) entre la zone promotrice et la zone codante ainsi qu'entre la zone codante et la zone terminatrice et qui peut être d'origine phylogénétique quelconque.

### EXEMPLE 1: Construction de gènes chimères

On effectue la construction de gènes chimères selon l'invention à partir des éléments suivants:
1) Promoteur de gène d'histone: on isole le promoteur à partir d'un clone d'histone H4A748 d'*Arabidopsis thaliana* race Strasbourg (M.E. Chaboute, Thèse de l'Université de Strasbourg, 1987 et M.E.Chaboute *et al.* (1987) Plant Mol.Biol., **8**, 179-191). Ce promoteur comprenant environ 900pb entre les sites Aval utilisés pour l'isoler a été sous-cloné au site Sall de pUC18 disponible sur catalogue (Pharmacia #27-4949-01) après remplissage des extrémités saillantes Sall et Aval par la polymérase de Klenow. L'un des sous-clones obtenu présentant l'extrémité 5' du promoteur proche du site Hindlll du polylinker de pUC18 et l'extrémité 3' du promoteur proche du site XbaI du polylinker de pUC18 a été utilisé par la suite et nommé: prom.H4A748/Aval/SalI-pUC18.
2) Zone peptide de transit: les deux peptides de transit ainsi que les éléments de protéines matures utilisés ont été décrits, de même que leur assemblage (M. Lebrun *et al*., demande de brevet européen 508 909). Cette zone comprend environ 350pb et est nommée Peptide de Transit Optimisé (PTO).
3) Gène de tolérance herbicide CT7: il provient du gène muté CT7 (Pro101 en Ser) de EPSPS de *Salmonella thyphymurium* isolé par Stalker *et al*. (1985) J.Biol.Chem., **260**, 4724-4728. Le clone pGM34-2, fourni par Calgene, a été linéarisé par Xbat puis traité par la nucléase de *Vigna radiata*. Après recoupure par Smal, les deux extrémités franches ont été ligaturées. Le clone obtenu possède un site Ncol sur l'ATG initiateur de la traduction, ainsi qu'un site Sall à 17pb en aval du codon stop. Ce clone a été nommé pRPA-BL-104.
4) Fusion TPO/CT7: une cassette d'expression contenant le transit peptide optimisé (TPO) fusionné dans le cadre de lecture avec le gène CT7 (B.Leroux *et al*., demande de brevet européen 507 698) est clonée dans le vecteur pBSII SK(-) (catalogue Stratagene #212206). Cette cassette 5'-TPO/CT7-3' contient un site Xbal à son extrémité 5' et un site SstI à son extrémité 3'.
5) Gène rapporteur GUS: il s'agit du gène codant pour la β-glucuronidase(GUS) présent dans le plasmide pBI 101-1 disponible sur catalogue (Clontech #6017-1).
6) Zone terminatrice histone: elle est isolée à partir du gène d'histone H4A748 d'*Arabidopsis thaliana* (M.E.Chaboute, thèse de l'Université de Strasbourg, 1987 et M.E.Chaboute *et al*. (1987) Plant Mol.Biol., **8**, 179-191). Un fragment Sau3AI de 661pb a été isolé, rendu à bords francs par traitement à la polymérase de Klenow et sous-cloné au site Smal du phagemide pBluescript® II KS (+) disponible sur catalogue (Stratagene #212207; Short et al., 1988, Nucleic Acid Research 16(15), 7583-7600). La séquence de ce fragment est présentée ci-dessous dans la section :"liste de séquence". Il a été montré par cartographie S1 que ce fragment isolé comprend à l'intérieur d'une région de 200 pb de son extrémité 5', dans le sens originel de la transcription, la séquence correspondant à celle présente avant le site de polyadénylation de l'ARNm dérivant de la transcription de ce gène. Le nucléotide A en position 165 de la séquence de 661pb correspondant au site d'addition de l'extrémité polyadénylée présente sur le transcrit (Chaboute M.E. *et al*. (1988) Gene, 71, 217-223). Deux sous-clones ont été conservés, qui présente l'un: l'insert orienté dans le sens originel de la transcription du gène H4A748 sous forme d'un fragment SstI-EcoRI et nommé t-directH4A748/Sau3Al/SmaI-pKS, et l'autre en sens inverse sous forme d'un fragment SstI-EcoRI et nommé t-inverseH4A748/Sau3Al/SmaI-pKS
   Séquence de 661pb de la zone terminatrice d'histone H4A748 d'*Arabidopsis thaliana* dans le sens originel de la transcription du gène: 
GATCCGCGTT TGTGTTTTCT GGGTTTCTCA CTTAAGCGTC TGCGTTTTAC TTTTGTATTG 60
GGTTTGGCGT TTAGTAGTTT GCGGTAGCGT TCTTGTTATG TGTAATTACG CTTTTTCTTC 120
TTGCTTCAGC AGTTTCGGTT GAAATATAAA TCGAATCAAG TTTCACTTTA TCAGCGTTGT 180
TTTAAATTTT GGCATTAAAT TGGTGAAAAT TGCTTCAATT TTGTATCTAA ATAGAAGAGA 240
CAACATGAAA TTCGACTTTT GACCTCAAAT CTTCGAACAT TTATTTCCTG ATTTCACGAT 300
GGATGAGGAT AACGAAAGGG CGGTTCCTAT GTCCGGGAAA GTTCCCGTAG AAGACAATGA 360
GCAAAGCTAC TGAAACGCGG ACACGACGTC GCATTGGTAC GGATATGAGT TAAACCGACT 420
CAATTCCTTT ATTAAGACAT AAACCGATTT TGGTTAAAGT GTAACAGTGA GCTGATATAA 480
AACCGAAACA AACCGGTACA AGTTTGATTG AGCAACTTGA TGACAAACTT CAGAATTTTG 540
GTTATTGAAT GAAAATCATA GTCTAATCGT AAAAAATGTA CAGAAGAAAA GCTAGAGCAG 600
AACAAAGATT CTATATTCTG GTTCCAATTT ATCATCGCTT TAACGTCCCT CAGATTTGAT 660
C 661
7) Zone terminatrice "nos": il s'agit du fragment contenant le signal de zone terminatrice du gène de la nopaline synthase (nos) de pTi37 (Bevan M. *et al*. (1983) Nucl.Acids Res., 11, 369-385) présent sur le plasmide pBI 101-1 disponible sur catalogue (Clontech #6017-1).

L'assemblage de tous ces éléments a été effectué de la façon suivante sur la base du squelette du vecteur binaire pour la transformation des plantes via *Agrobacterium tumefaciens*, pBI 101-1 disponible sur catalogue (Clontech #6017-1):

### Construction de pRA-1:

Le promoteur histone H4A748 d'*Arabidopsis thaliana* a été excisé du plasmide prom.H4A748/Aval/Sall-pUC18 sous forme du fragment HindIII/XbaI de environ 900pb. Ce fragment a été inséré par ligation dans le plasmide pBI 101-1 digéré par HindIII/XbaI. Le plasmide recombinant obtenu contient, dans le sens de la transcription du gène chimère: promoteur H4A748/GUS/zone terminatrice nos et a été nommé pRA-1.

### Construction de pRA-2:

Le zone terminatrice H4A748 d'*Arabidopsis thaliana* a été excisé du plasmide t-directH4A748/Sau3Al/SmaI-pKS sous forme d'un fragment SstI/EcoRI d'environ 670pb. Ce fragment a été inséré par ligation en substitution de la zone terminatrice nos après digestion du plasmide pRA-1 par SstI/EcoRI. Le plasmide recombinant obtenu contient, dans le sens de la transcription du gène chimère: promoteur H4A748/GUS/zone terminatrice H4A748 en orientation directe et a été nommé pRA-2.

### Construction de pRA-3:

Le zone terminatrice H4A748 d'*Arabidopsis thaliana* a été excisé du plasmide 1-inverseH4A748/Sau3Al/SmaI-pKS sous forme d'un fragment SstI/EcoRI d'environ 670pb. Ce fragment a été inséré par ligation en substitution de la zone terminatrice nos après digestion du plasmide pRA-1 par Sst1/EcoRI. Le plasmide recombinant obtenu contient, dans le sens de la transcription du gène chimère: promoteur H4A748/GUS/zone terminatrice H4A748 en orientation inverse et a été nommé pRA-3.

### Construction de pRPA-RD-146:

Le fragment XbaI/SstI d'environ 1,75kpb contenant PTO/CT7 a été inséré par ligation en substitution du gène GUS après digestion du plasmide pRA-1 par XbaI/SstI. Le plasmide recombinant obtenu contient, dans le sens de la transcription du gène chimère: promoteur H4A748/PTO/CT7/zone terminatrice nos et a été nommé pRPA-RD-146.

### Construction de pRPA-RD-147:

Le fragment XbaI/SstI d'environ 1,75kpb contenant PTO/CT7 a été inséré par ligation en substitution du gène GUS après digestion du plasmide pRA-2 par XbaI/SstI. Le plasmide recombinant obtenu contient, dans le sens de la transcription du gène chimère: promoteur H4A748/PTO/CT7/zone terminatrice H4A748 en orientation directe et a été nommé pRPA-RD-147.

### Construction de pRPA-RD-148:

Le fragment XbaI/SstI d'environ 1,75kpb contenant PTO/CT7 a été inséré par ligation en substitution du gène GUS après digestion du plasmide pRA-3 par XbaI/SstI. Le plasmide recombinant obtenu contient, dans le sens de la transcription du gène chimère: promoteur H4A748/PTO/CT7/zone terminatrice H4A748 en orientation inverse et a été nommé pRPA-RD-148.

### EXEMPLE 2: Expression de l'activité d'un gène rapporteur

### 1) Transformation et régénération

Le vecteur est introduit dans la souche non oncogène d'*Agrobacterium tumefaciens* LBA 4404 disponible sur catalogue (Clontech #6027-1) par croisement triparental à l'aide du plasmide "helper" pRK 2013 dans *Escherichia coli* HB101 selon la procédure décrite par Bevan M. (1984) Nucl.Acids Res., 12, 8711-8721.

La technique de transformation à partir d'explants racinaires d'*Arabidopsis thaliana* L.-écotype C24 a été effectuée selon la procédure décrite par Valvekens D. et al. (1988) Proc.Natl.Acad.Sci USA, 85, 5536-5540. Brièvement, 3 étapes sont nécessaires: induction de la formation des cals sur milieu B5 de Gamborg complémenté de 2,4-D et de kinétine; formation de bourgeons sur milieu B5 de Gamborg complémenté de 2iP et d'IAA; enracinement et formation de graines sur MS sans hormones.

### 2) Mesure de l'activité GUS dans les plantes

### a- observations histochimiques

La révélation de l'activité GUS (Jefferson R.A. et al. (1987) EMBO J., **6**, 3901-3907) sur différents organes de plantes transgéniques montre que l'expression préférentielle dans les régions méristématiques est conservée avec les constructions pRA-2 et 3. De plus, la construction pRA-2 permet d'observer une activité dans les tissus adultes (feuilles, racines, hampes florales) qui n'apparaît pas avec la construction pRA-1.

### b- mesures fluorométriques

L'activité GUS a été mesurée par fluorométrie sur des extraits de bourgeons floraux et de feuilles de la rosette (Jefferson R.A. et al. (1987) EMBO J., **6**, 3901-3907) à partir de 12 plantes, correspondant à des évènements individuels de transformation, pour chacune des constructions pRA-1, 2 et 3.
Les résultats moyens calculés, en pmol MU/min.mg prot., sont les suivants:

| Constructions | Feuilles (F) | Bourgeons (B) | Rapports B/F |
|---|---|---|---|
| pRA-1 | 850,25 | 3965,33 | 4,66 |
| pRA-2 | 4890,67 | 33683,75 | 6,89 |
| pRA-3 | 4211,73 | 27752,45 | 6,59 |

Ces mesures montrent clairement que la zone terminatrice H4A748 en sens direct ou inverse induit une augmentation de l'activité de l'expression du gène chimère, en particulier dans les régions de la plante en croissance rapide.

### EXEMPLE 3: Tolérance de plantes transgéniques à un herbicide

### 1) Transformation et régénération

Le vecteur est introduit dans la souche non oncogène d'*Agrobacterium tumefaciens* LBA 4404 disponible sur catalogue (Clontech #6027-1) par croisement triparental à l'aide du plasmide "helper" pRK 2013 dans *Escherichia coli* HB101 selon la procédure décrite par Bevan M. (1984) Nucl.Acids Res., **12**, 8711-8721.

La technique de transformation à partir d'explants foliaires de tabac est basée sur la procédure décrite par Horsh R. et al. (1985) Science, **227**, 1229-1231. La régénération du tabac PBD6 (provenance SEITA-France) à partir d'explants foliaires est réalisée sur un milieu de base Murashige et Skoog (MS) comprenant 30g/l de saccharose ainsi que 200□g/ml de kanamycine en trois étapes successives: la première comprend l'induction des pousses sur un milieu MS additionné de 30g de saccharose contenant 0,05mg d'acide naphtylacétique (ANA) et 2mg/l de benzylaminopurine (BAP) pendant 15 jours. Les pousses formées au cours de cette étape sont ensuite développées par culture sur un milieu MS additionné de 30g/l de saccharose mais ne contenant pas d'hormone, pendant 10 jours. Puis on prélève des pousses développées et on les cultive sur un milieu d'enracinement MS dilué au demi, à teneur moitié en sels, vitamines et sucres et ne contenant pas d'hormone. Au bout d'environ 15 jours, les pousses enracinées sont passées en terre.

### 2) Mesure de la tolérance au glyphosate:

Vingt plantes transformées ont été régénérées et passées en serre pour chacune des constructions pRPA-RD-A, B et C. Ces plantes ont été traitées en serre au stade 5 feuilles avec une suspension aqueuse de Roundup® correspondant à 0,8 kg de matière active glyphosate par hectare.
Les résultats correspondent à l'observation d'indices de phytotoxicité relevés 3 semaine après traitement. Dans ces conditions, on constate que les plantes transformées par les constructions présentent en moyenne une tolérance acceptable (pRPA-RD-146) voire bonne (pRPA-RD-147 et 148) alors que les plantes témoins non transformées sont complètement détruites.
Ces résultats montrent clairement l'amélioration apportée par l'utilisation d'un gène chimère selon l'invention pour un même gène codant pour la tolérance au glyphosate.

Les plantes transformées selon l'invention peuvent être utilisées comme parents pour l'obtention de lignées et d'hybrides ayant le caractère phénotypique correspondant à l'expression du gène chimère introduit.

## Revendications

1. Gène chimère comprenant dans le sens de la transcription une zone promotrice, une séquence codant pour une protéine d'intérêt agronomique et une zone terminatrice, **caractérisé en ce que** la zone terminatrice est une zone terminatrice d'un gène d'histone végétal.

2. Gène chimère selon la revendication 1, **caractérisé en ce que** la zone terminatrice est orientée, dans le sens de la transcription du gène chimère, de façon directe ou inversée relativement à son orientation initiale dans le sens de la transcription du gène dont elle est issue.

3. Gène chimère selon l'une des revendications 1 et 2, **caractérisé en ce que** le zone terminatrice d'histone provient d'une plante dicotylédone.

4. Gène chimère selon la revendication 3, **caractérisé en ce que** le zone terminatrice d'histone provient d'*Arabidopsis thaliana.*

5. Gène chimère selon l'une des revendications 1 et 2, **caractérisé en ce que** le zone terminatrice d'histone provient d'une plante monocotylédone.

6. Gène chimère selon la revendication 5, **caractérisé en ce que** le zone terminatrice d'histone provient de *Zea mays*.

7. Gène chimère selon l'une des revendications 1 à 6, **caractérisé en ce que** le zone terminatrice d'histone provient d'un gène d'histone végétale H4.

8. Gène chimère selon l'une des revendications 1 à 6, **caractérisé en ce que** le zone terminatrice d'histone provient d'un gène d'histone végétale H3.

9. Gène chimère selon l'une des revendications 1 à 8, **caractérisé en ce que** la zone promotrice provient d'un promoteur de gène d'histone végétale.

10. Gène chimère selon la revendication 9, **caractérisé en ce que** la zone promotrice provient du même gène d'histone végétale que le zone terminatrice.

11. Gène chimère selon l'une des revendications 9 ou 10, **caractérisé en ce que** la zone promotrice comprend un promoteur d'histone végétale dupliqué.

12. Gène chimère selon l'une des revendications 9 ou 10, **caractérisé en ce que** la zone promotrice contient au moins une zone promotrice d'un gène d'histone végétale associée à une zone promotrice différente issue d'un gène pouvant s'exprimer naturellement dans les plantes.

13. Gène chimère selon l'une des revendications 1 à 12, **caractérisé en ce que** la séquence codante permet de conférer aux plantes une tolérance accrue vis à vis d'un herbicide.

14. Gène chimère selon la revendication 13, **caractérisé en ce que** le séquence codante conférant une tolérance herbicide est fusionnée à une séquence ADN codant pour une zone peptide de transit permettant l'accumulation du produit de la traduction du gène de tolérance herbicide dans un compartiment subcellulaire.

15. Gène chimère selon la revendication 14, **caractérisé en ce que** la zone peptide de transit permet l'accumulation du produit de la traduction du gène de tolérance herbicide dans le compartiment plastidial.

16. Gène chimère selon la revendication 15, **caractérisé en ce que** la zone peptide de transit comprend, dans le sens de la transcription, au moins un peptide de transit d'un gène végétal codant pour une enzyme à localisation plastidiale, éventuellement une partie de séquence de la partie mature N-terminale d'un gène végétal codant pour une enzyme à localisation plastidiale, puis éventuellement un second peptide de transit d'un gène végétal codant pour une enzyme à localisation plastidiale.

17. Gène chimère selon l'une des revendications 13 à 16, **caractérisé en ce que** le gène de tolérance herbicide code pour une enzyme active vis à vis d'herbicides dont la cible est l'EPSPS.

18. Gène chimère selon la revendication 17, **caractérisé en ce que** le gène de tolérance herbicide code pour une enzyme active vis à vis du glyphosate.

19. Gène chimère selon la revendication 18, **caractérisé en ce que** le gène de tolérance herbicide est d'origine phylogénétique quelconque.

20. Vecteur pour la transformation des plantes, **caractérisé en ce qu'**il comprend un gène chimère selon l'une des revendications 1 à 19.

21. Souche d'*Agrobacterium* sp., **caractérisée en ce qu'**elle contient un vecteur selon la revendication 20.

22. Cellule végétale transformée, **caractérisée en ce qu'**elle contient un gène chimère selon l'une des revendications 1 à 19.

23. Plante transformée obtenue à partir d'une cellule selon la revendication 22, ladite plante contenant le gène selon les revendications 1 à 19 ou le vecteur selon la revendication 20.

## Patentansprüche

1. Chimäres Gen, das in Transkriptionsrichtung eine Promoterregion, eine Sequenz, die für ein interessierendes agronomisches Protein codiert, und eine Terminatorregion umfaßt, **dadurch gekennzeichnet, daß** es sich bei der Terminatorregion um eine Terminatorregion eines pflanzlichen Histon-Gens handelt.

2. Chimäres Gen nach Anspruch 1, dadurch gekennzesichnet, daß die Terminatorregion in Transkriptionsrichtung des chimären Gens in gleicher oder umgekehrter Richtung in Bezug auf seine ursprüngliche Orientierung in Transkriptionsrichtung des Gens, von dem sie stammt, vorliegt.

3. Chimäres Gen nach Anspruch 1 oder 2, **dadurch gekennzeichnet, daß** die Histon-Terminatorregion von einer dikotylen Pflanze stammt.

4. Chimäres Gen nach Anspruch 3, **dadurch gekennzeichnet, daß** die Histon-Terminatorregion von *Arabidopsis thaliana* stammt.

5. Chimäres Gen nach Anspruch 1 oder 2, **dadurch gekennzeichnet, daß** die Histon-Terminatorregion von einer monokotylen Pflanze stammt.

6. Chimäres Gen nach Anspruch 5, **dadurch gekennzeichnet, daß** die Histon-Terminatorregion von *Zea mays* stammt.

7. Chimäres Gen nach einem der Ansprüche 1 bis 6, **dadurch gekennzeichnet, daß** die Histon-Terminatorregion von einem pflanzlichen Histon-Gen H4 stammt.

8. Chimäres Gen nach einem der Ansprüche 1 bis 6, **dadurch gekennzeichnet, daß** die Histon-Terminatorregion von einem pflanzlichen Histon-Gen H3 stammt.

9. Chimäres Gen nach einem der Ansprüche 1 bis 8, **dadurch gekennzeichnet, daß** die Promoterregion von einem Promoter eines pflanzlichen Histon-Gens stammt.

10. Chimäres Gen nach Anspruch 9, **dadurch gekennzeichnet, daß** die Promoterregion von dem gleichen pflanzlichen Histon-Gen wie die Terminatorregion stammt.

11. Chimäres Gen nach Anspruch 9 oder 10, **dadurch gekennzeichnet, daß** die Promoterregion einen verdoppelten pflanzlichen Histon-Promoter umfaßt.

12. Chimäres Gen nach Anspruch 9 oder 10, **dadurch gekennzeichnet, daß** die Promoterregion mindestens eine Promoterregion von einem pflanzlichen Histon-Gen in Kombination mit einer unterschiedlichen Promoterregion, die von einem Gen, das natürlich in Pflanzen exprimiert werden kann, stammt, enthält.

13. Chimäres Gen nach einem der Ansprüche 1 bis 12, **dadurch gekennzeichnet, daß** man mit der Codiersequenz Pflanzen eine erhöhte Toleranz gegenüber einem Herbizid zu verleihen vermag.

14. Chimäres Gen nach Anspruch 13, **dadurch gekennzeichnet, daß** die Codiersequenz, die eine Herbizidtoleranz verleiht, mit einer DNA-Sequenz für eine Transitpeptidregion, die eine Anhäufung des Translationsprodukts des Herbizidtoleranzgens in einem subzellulären Kompartiment gestattet, fusioniert ist.

15. Chimäres Gen nach Anspruch 14, **dadurch gekennzeichnet, daß** die Transitpeptidregion die Anhäufung des Translationsprodukts des Herbizidtoleranzgens im Kompartiment der Plastiden gestattet.

16. Chimäres Gen nach Anspruch 15, **dadurch gekennzeichnet, daß** die Transitpeptidregion in Transkriptionsrichtung mindestens ein Transitpeptid eines pflanzlichen Gens, das für ein plastidär lokalisiertes Enzym codiert, ggf. einen Teil der Sequenz des reifen n-terminalen Abschnitts eines pflanzlichen Gens, das für ein plastidär lokalisiertes Enzym codiert, sowie ggf. ein zweites Transitpeptid eines pflanzlichen Gens, das für ein plastidär lokalisiertes Enzym codiert, umfaßt.

17. Chimäres Gen nach einem der Ansprüche 13 bis 16, **dadurch gekennzeichnet, daß** das Herbizidtoleranzgen für ein Enzym, das gegen Herbizide, deren Angriffspunkt die EPSPS ist, aktiv sind, codiert.

18. Chimäres Gen nach Anspruch 17, **dadurch gekennzeichnet, daß** das Herbizidtoleranzgen für ein Enzym, das gegen Glyphosate aktiv ist, codiert.

19. Chimäres Gen nach Anspruch 18, **dadurch gekennzeichnet, daß** das Herbizidtoleranzgen beliebigen phylogenetischen Ursprungs ist.

20. Vektor für die Transformation von Pflanzen, **dadurch gekennzeichnet, daß** er ein chimäres Gen nach einem der Ansprüche 1 bis 19 umfaßt.

21. *Agrobacterium* sp. Stamm, **dadurch gekennzeichnet, daß** er einen Vektor nach Anspruch 20 enthält.

22. Transformierte Pflanzenzelle, **dadurch gekennzeichnet, daß** sie ein chimäres Gen nach einem der Ansprüche 1 bis 19 enthält.

23. Transformierte Pflanze, die von einer Zelle nach Anspruch 22 stammt, wobei diese Pflanze das Gen nach den Ansprüchen 1 bis 19 oder den Vektor nach Anspruch 20 enthält.

## Claims

1. Chimeric gene comprising, in the direction of transcription, a promoter region, a sequence encoding a protein of agronomic interest and a terminator region, **characterized in that** the terminator region is a terminator region of a plant histone gene.

2. Chimeric gene according to Claim 1, **characterized in that** the terminator region is oriented, in the direction of transcription of the chimeric gene, in a direct or reversed manner relative to its initial orientation in the direction of transcription of the gene from which it is derived.

3. Chimeric gene according to either of Claims 1 and 2, **characterized in that** the histone terminator region is obtained from a dicotyledonous plant.

4. Chimeric gene according to Claim 3, **characterized in that** the histone terminator region is obtained from *Arabidopsis thaliana*.

5. Chimeric gene according to either of Claims 1 and 2, **characterized in that** the histone terminator region is obtained from a monocotyledonous plant.

6. Chimeric gene according to Claim 5, **characterized in that** the histone terminator region is obtained from *Zea mays*.

7. Chimeric gene according to one of Claims 1 to 6, **characterized in that** the histone terminator region is obtained from a plant histone gene H4.

8. Chimeric gene according to one of Claims 1 to 6, **characterized in that** the histone terminator region is obtained from a plant histone H3.

9. Chimeric gene according to one of Claims 1 to 8, **characterized in that** the promoter region is obtained from a plant histone gene promoter.

10. Chimeric gene according to Claim 9, **characterized in that** the promoter region is obtained from the same plant histone gene as the terminator region.

11. Chimeric gene according to either of Claims 9 and 10, **characterized in that** the promoter region comprises a duplicated plant histone promoter.

12. Chimeric gene according to either of Claims 9 and 10, **characterized in that** the promoter region contains at least one promoter region of a plant histone gene combined with a different promoter region derived from a gene which can be expressed naturally in plants.

13. Chimeric gene according to one of Claims 1 to 12, **characterized in that** the coding sequence makes it possible to confer on plants increased tolerance to a herbicide.

14. Chimeric gene according to Claim 13, **characterized in that** the coding sequence conferring herbicide tolerance is fused with a DNA sequence encoding a transit peptide region allowing accumulation of the product of translation of the herbicide tolerance gene in a subcellular compartment.

15. Chimeric gene according to Claim 14, **characterized in that** the transit peptide region allows accumulation of the product of translation of the herbicide tolerance gene in the plastid compartment.

16. Chimeric gene according to Claim 15, **characterized in that** the transit peptide region comprises, in the direction of transcription, at least one transit peptide from a plant gene encoding a plastid-localized enzyme, optionally part of a sequence of the N-terminal mature part of a plant gene encoding a plastid-localized enzyme, and then optionally a second transit peptide from a plant gene encoding a plastid-localized enzyme.

17. Chimeric gene according to one of Claims 13 to 16, **characterized in that** the herbicide tolerance gene encodes an enzyme which is active towards herbicides whose target is EPSPS.

18. Chimeric gene according to Claim 17, **characterized in that** the herbicide tolerance gene encodes an enzyme which is active towards glyphosate.

19. Chimeric gene according to Claim 18, **characterized in that** the herbicide tolerance gene is of any phylogenetic origin.

20. Vector for transforming plants, **characterized in that** it comprises a chimeric gene according to one of Claims 1 to 19.

21. Strain of *Agrobacterium* sp., **characterized in that** it contains a vector according to Claim 20.

22. Transformed plant cell, **characterized in that** it contains a chimeric gene according to one of Claims 1 to 19.

23. Transformed plant obtained from a cell according to Claim 22, the said plant containing the gene according to Claims 1 to 19 or the vector according to Claim 20.
